# EUROPEAN PATENT APPLICATION

(11) **EP 3 388 008 A1**
(43) Date of publication of application: **17.10.2018**
(21) Application number: 16918568.3
(22) Date of filing: 29.12.2016
(51) Int. Cl.: A61B 17/80, A61B 17/84

(54) **ABSORBABLE SKULL FIXATION LOCK SYSTEM AND METHOD FOR USING SAME**

(30) Priority: 13.10.2016 CN 201610891192; 15.12.2016 CN 201611157582
(71) Applicant: Suzhou Ship Biological Technology Co., Ltd., Suzhou, Jiangsu 200127 (CN)
(72) Inventor: GE, Jianwei, Suzhou Jiangsu 200127 (CN); GE, Liya, Suzhou Jiangsu 200127 (CN); GE, Lijin, Suzhou Jiangsu 200127 (CN); GE, Liyu, Suzhou Jiangsu 200127 (CN)
(74) Representative: Hoffmann Eitle
(86) International application number: PCT/CN2016/112894
(87) International publication number: WO 2018/068410

(57) **Abstract**

The present invention relates to the technical field of medical instruments, and in particular, to an absorbable skull fixing lock system and a method of using same. The absorbable skull fixing lock system includes an absorbable skull fixing lock and a locker. The absorbable skull fixing lock system may be used by means of correct implantation of the absorbable skull fixing lock, and pre-locking and final locking of the absorbable skull fixing lock. The present invention is easy to operate and needs no additional instrument in a skull fixation operation; the present invention is quick and timesaving, and thus reduces the operation time; due to the elliptical design of a locking surface, the locking surface is more reliable and safer; due to the toothed design of the locking surface, no shift occurs, so that a lock body is safer and does not slip off; by means of a fixing pin, it is ensured that two lock plates can be center-aligned when the two lock plates are locked, and the alignment is good; a force limiting valve is designed to ensure that the lock body is safer, and ensure that the locking force applied to each lock body by a doctor when locking the fixing lock is the same.

## Description

The present invention relates to the technical field of medical instruments, and in particular, to an absorbable skull fixing lock system and a method of using same.

### RELATED ART

Skull fixation is an essential process in a neurosurgical operation. Because nails and plates commonly used in the existing skull fixation methods are generally made of titanium alloy, both drilling and tapping operations are required in the surgical procedure and are tedious and time-consuming. Nevertheless, absorbable nails and plates, which are easily broken due to small screws, cause surgery failure.

### SUMMARY

To solve the deficiencies of the prior art, the present invention aims to provide an absorbable skull fixing lock system and a method of using same, which can fix skull safely, reliably and conveniently.

In order to achieve the objective above, the present invention adopts the following technical solutions:
The present invention provides an absorbable skull fixing lock system, comprising two parts, i.e., an absorbable skull fixing lock and a locker. The absorbable skull fixing lock comprises an upper implant lock plate, a lower implant lock plate, a fixing pin, and a locking wire; the locker comprises a locking handle, a force limiting valve connected to the locking handle, a knot pusher connected to the force limiting valve, and a knotter connected to the knot pusher; an edge of the upper implant lock plate, the knotter, the knot pusher, the force limiting valve, and the locking handle are connected in sequence; the locking wire extends out of the force limiting valve, passes through the knot pusher and the knotter and is wound on the upper implant lock plate and the lower implant lock plate.

The fixing pin matches the lower implant lock plate by means of close fit and then is inserted into two holes of the upper implant lock plate; the upper implant lock plate may slide along the fixing pin; the fixing pin is connected to an inverted tapered pin hole of the lower implant lock plate by means of interference fit.

The locking handle is a T-shaped locking handle. Both the upper implant lock plate and the lower implant lock plate are elliptical and toothed. The lower implant lock plate and the fixing pin are connected using an inverted tapered hole by means of interference fit. The upper implant lock plate and the lower implant lock plate are positioned by means of the fixing pin; the fixing pin has positioning and locking functions.

According to the absorbable skull fixing lock system, (1) the absorption time that the lock plates and the locking wire can be absorbed is 12 to 16 months; (2) the system is easy to operate and needs no additional instrument in a skull fixation operation; (3) the system is quick and timesaving, and thus reduces the operation time; (4) due to the elliptical design of a locking surface of a locking body, the locking surface is more reliable and safer; (5) due to the toothed design of the locking surface, no shift occurs, so that the lock body is safer and does not slip off; (6) by means of the fixing pin, it is ensured that the two lock plates can be center-aligned when the two lock plates are locked, and the alignment is good; (7) the fixing pin is designed to have positioning and locking functions; and (8) the force limiting valve is designed to ensure that the lock body is safer, and ensure that the locking force applied to each lock body by a doctor when locking the fixing lock is the same.

A method of using an absorbable skull fixing lock system, comprising the following steps:
I, correct implantation of absorbable skull fixing locks
   step 1: taking out each component of an absorbable skull fixing lock system in a surgical sterile environment;
   step 2: opening upper and lower implant lock plates and straightening out a locking wire for future use;
   step 3: holding a fixing pin with a hand, placing the lower implant lock plate between a bone flap and a dura mater, and ensuring that a protruding positioning block of the lower implant lock plate is between skull sutures and is in close contact with the edge of the bone flap;
   step 4: placing the locking handle outside an open area of the skull and at least a plurality of absorbable skull fixing locks between the skull sutures;
   step 5: putting the bone flap back to an initial position, and confirming that the position of the lower implant lock plate is still in a bone flap suture and is not shifted; and
   step 6: gently pulling a knot of the locking wire after the upper implant lock plate is well placed, grasping the knotted locking wire, and pressing the bone flap with the other hand to prevent same from shifting;
II, pre-locking of the absorbable skull fixing locks
   step 1: releasing a knot pusher from the locking handle, and slowly pushing the knot pusher to the edge of the upper implant lock plate;
   step 2: pushing the knot pusher, and pulling the locking handle straight down to avoid cutting the suture; and
   step 3: not rushing to snap the locking wire, so that all the absorbable skull fixing locks are pre-locked;
III, final locking of the absorbable skull fixing locks
   step 1: gripping the knot pusher to closely attach same to the upper implant lock plate, and pulling the handle until a force limiting valve falls off automatically, so that the bone flap is well fixed with the skull fixing locks at the optimum strength at this time;
   step 2: if a completely absorbable locking wire is employed, reserving a 2-3 mm portion of the fixing pin protruding out of the upper implant lock plate and then cutting away the remaining portion, and ironing the reserved portion by using an instrument (due to the inverted tapered design of an upper lock plate fixing pin hole, ironing is implemented by heating to 60 degrees centigrade, so that enough locking force is achieved after the ironing, and the ironing refers to making the fixing pin not protrude out of the arc surface of the upper implant lock plate, i.e., may be an elliptical surface of the upper implant lock plate, the arc surface of the upper implant lock plate is flush with the end of the fixing pin, and the fixing pin is melted at the inverted tapered hole of the upper implant lock plate by means of ironing to achieve the function of connecting and fixing the upper implant locking plate); when a non-absorbable locking wire is employed, cutting away the portion of the fixing pin protruding out of the upper implant lock plate to be parallel to the arc surface of the upper implant lock plate, that is, the fixing pin is flush with the arc surface of the upper implant lock plate;
   step 3: repeating the operations of steps 1 and 2 for all the absorbable skull fixing locks.

The present invention is beneficial in that: 1, the locking approach of the lock plates is simple and reliable; 2, the force limiting valve ensures that all the locking forces are the same; 3, it is ensured that all skull fixing implant materials are designed to be degradable materials; 4, a series of drilling and tapping operations in the existing skull fixation methods are avoided; and 5, the present invention is easy to use and can be proficiently operated only through simple training.

### BRIEF DESCRIPTION OF THE DRAWINGS

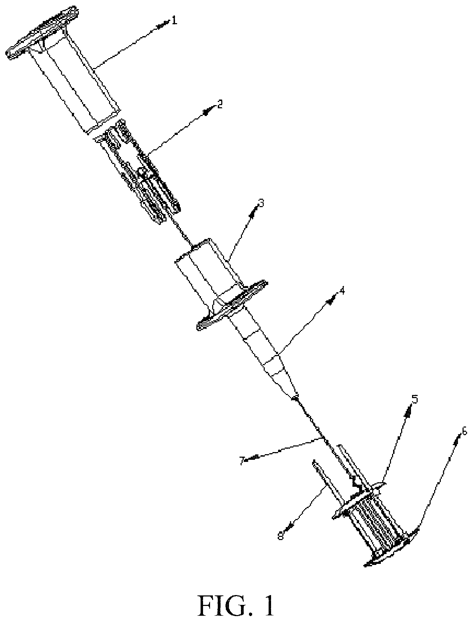

FIG. 1 is a structural diagram of an absorbable skull fixing lock system of the present invention.The meanings of the reference numerals in the drawing are as follows:
1 T-shaped locking handle; 2 force limiting valve; 3 knot pusher; 4 knotter; 5 upper implant lock plate; 6 lower implant lock plate; 7 locking wire; 8 fixing pin.

### DETAILED DESCRIPTION

The present invention is described in detail below in combination with the accompanying drawings and specific embodiments.

According to FIGs. 1 and 2, an absorbable skull fixing lock system comprises an absorbable skull fixing lock and a locker, wherein the absorbable skull fixing lock comprises an upper implant lock plate 5, a lower implant lock plate 6, a locking wire 7, and a fixing pin 8; the locker comprises a T-shaped locking handle 1, a force limiting valve 2 connected to the locking handle, a knot pusher 3 connected to the force limiting valve, and a knotter 4 connected to the knot pusher; an edge of the upper implant lock plate, the knotter, the knot pusher, the force limiting valve, and the locking handle are connected in sequence; the locking wire extends out of the force limiting valve, passes through the knot pusher and the knotter and is wound on the upper implant lock plate and the lower implant lock plate; the fixing pin is in inverted tapered interference fit with the lower implant lock plate and is in sliding fit with the upper implant lock plate.

Both the upper implant lock plate and the lower implant lock plate are elliptical and toothed.

A method of using an absorbable skull fixing lock system comprises the following steps:
I, correct implantation of absorbable skull fixing locks
   step 1: take out each component of an absorbable skull fixing lock system in a surgical sterile environment;
   step 2: open upper and lower implant lock plates and straighten out a locking wire for future use;
   step 3: hold a fixing pin with a hand, place the lower implant lock plate between a bone flap and a dura mater, and ensure that a protruding positioning block of the lower implant lock plate is between skull sutures and is in close contact with the edge of the bone flap;
   step 4: place the locking handle outside an open area of the skull and at least a plurality of absorbable skull fixing locks between the skull sutures;
   step 5: put the bone flap back to an initial position, and confirm that the position of the lower implant lock plate is still in a bone flap suture and is not shifted;
   step 6: gently pull a knot of the locking wire after the upper implant lock plate is well placed, grasp the knotted locking wire, and press the bone flap with the other hand to prevent same from shifting;
II, pre-locking of the absorbable skull fixing locks
   step 1: release a knot pusher from the locking handle, and slowly push the knot pusher to the edge of the upper implant lock plate;
   step 2: push the knot pusher, and pull the locking handle straight down to avoid cutting the suture;
   step 3: not rush to snap the locking wire, so that all the absorbable skull fixing locks are pre-locked;
III, final locking of the absorbable skull fixing locks
   step 1: grip the knot pusher to closely attach same to the upper implant lock plate, and first pull the handle until a force limiting valve falls off automatically, so that the bone flap is well fixed with the skull fixing locks at the optimum strength at this time;
   step 2: if a completely absorbable locking wire is employed, reserve a 2-3 mm portion of the fixing pin protruding out of the upper implant lock plate and then cut away the remaining portion, and iron the reserved portion by using an instrument (due to the inverted tapered design of an upper lock plate fixing pin hole, enough locking force is achieved after ironing); when a non-absorbable locking wire is used, cut away the portion of the fixing pin protruding out of the upper implant lock plate so that the fixing pin is flush with the arc surface of the upper implant lock plate;
   step 3: repeat the operations of steps 1 and 2 for all the absorbable skull fixing locks.

Preferably, at least three absorbable skull fixing locks are placed between the skull sutures and are put into an equilateral triangle.

The above illustrates and describes basic principles, main features and advantages of the present invention. Those skilled in the art should understand that the above descriptions are not intended to limit the present invention in any form, and all technical solutions obtained by employing equivalent substitutions or equivalent variations fall within the scope of protection of the present invention.

## Claims

1. An absorbable skull fixing lock system, comprising an absorbable skull fixing lock and a locker, wherein the absorbable skull fixing lock comprises an upper implant lock plate, a lower implant lock plate, a fixing pin, and a locking wire; the locker comprises a locking handle, a force limiting valve connected to the locking handle, a knot pusher connected to the force limiting valve, and a knotter connected to the knot pusher; an edge of the upper implant lock plate, the knotter, the knot pusher, the force limiting valve, and the locking handle are connected in sequence; the locking wire is connected to the force limiting valve by means of knotting, passes through the knot pusher and the knotter and is wound on the upper implant lock plate and the lower implant lock plate.

2. The absorbable skull fixing lock system according to claim 1, wherein the fixing pin matches the lower implant lock plate by means of close fit and then is inserted into two holes of the upper implant lock plate; the upper implant lock plate may slide along the fixing pin; the fixing pin is connected to an inverted tapered pin hole of the lower implant lock plate by means of interference fit.

3. The absorbable skull fixing lock system according to claim 1, wherein the locking handle is a T-shaped locking handle.

4. The absorbable skull fixing lock system according to claim 1, wherein both the upper implant lock plate and the lower implant lock plate are elliptical and toothed.

5. The absorbable skull fixing lock system according to claim 1, wherein the lower implant lock plate and the fixing pin are connected using an inverted tapered hole by means of interference fit.

6. The absorbable skull fixing lock system according to claim 1, wherein the upper implant lock plate and the lower implant lock plate are positioned by means of the fixing pin.

7. A method of using an absorbable skull fixing lock system, comprising the following steps:
I, correct implantation of absorbable skull fixing locks
step 1: taking out each component of the absorbable skull fixing lock system in a surgical sterile environment;
step 2: opening upper and lower implant lock plates and straightening out a locking wire for future use;
step 3: holding a fixing pin with a hand, placing the lower implant lock plate between a bone flap and a dura mater, and ensuring that a protruding positioning block of the lower implant lock plate is between skull sutures and is in close contact with the edge of the bone flap;
step 4: placing the locking handle outside an open area of the skull and at least a plurality of absorbable skull fixing locks between the skull sutures;
step 5: putting the bone flap back to an initial position, and confirming that the position of the lower implant lock plate is still in a bone flap suture and is not shifted; and
step 6: gently pulling a knot of the locking wire after the upper implant lock plate is well placed, grasping the knotted locking wire, and pressing the bone flap with the other hand to prevent same from shifting;
II, pre-locking of the absorbable skull fixing locks
step 1: releasing a knot pusher from the locking handle, and slowly pushing the knot pusher to the edge of the upper implant lock plate;
step 2: pushing the knot pusher, and pulling the locking handle straight down to avoid cutting the suture; and
step 3: not rushing to snap the locking wire, so that all the absorbable skull fixing locks are pre-locked;
III, final locking of the absorbable skull fixing locks
step 1: gripping the knot pusher to closely attach same to the upper implant lock plate, and pulling the handle until a force limiting valve falls off automatically, so that the bone flap is well fixed with the skull fixing locks at the optimum strength at this time;
step 2: when a completely absorbable locking wire is employed, reserving a 2-3 mm portion of the fixing pin protruding out of the upper implant lock plate and then cutting away the remaining portion, and ironing the reserved portion; when a non-absorbable locking wire is employed, cutting away the portion of the fixing pin protruding out of the upper implant lock plate, that is, the fixing pin is flush with the arc surface of the upper implant lock plate; and
step 3: repeating the operations of steps 1 and 2 for all the absorbable skull fixing locks.

8. The method of using an absorbable skull fixing lock system according to claim 7, wherein at least three absorbable skull fixing locks are placed between the skull sutures and are put into an equilateral triangle.
